# EUROPEAN PATENT APPLICATION

(11) **EP 2 428 228 A1**
(43) Date of publication of application: **14.03.2012**
(21) Application number: 10195929.4
(22) Date of filing: 20.12.2010
(51) Int. Cl.: A61K 48/00

(54) **Use of a linear DNA expression construct with covalently closed ends**

(30) Priority: 08.09.2010 GB 1014907
(71) Applicant: Max Delbrück Centrum für Molekulare Medizin (MDC) Berlin-Buch, 13125 Berlin (DE)
(72) Inventor: Walther, Wolfgang, 16341 Panketal (DE); Kobelt, Dennis, 13189 Berlin (DE); Schlag, Peter M., 13467 Berlin (DE)
(74) Representative: Krauss, Jan

(57) **Abstract**

The present invention relates to a linear DNA expression construct having covalently closed ends, comprising an expression cassette comprising a coding sequence encoding for a gene therapy anti-cancer agent for use in the treatment of cancer, wherein said DNA expression construct is suitable for administration into cells or tissues by a physical transfection method. Preferably, said DNA expression construct consists of a dumbbell-shaped circular strand of DNA.

## Description

The present invention relates to a linear DNA expression construct having covalently closed ends, comprising an expression cassette comprising a coding sequence encoding for a gene therapy anti-cancer agent for use in the treatment of cancer, wherein said DNA expression construct is suitable for administration into cells or tissues by a physical transfection method. Preferably, said DNA expression construct consists of a dumbbell-shaped circular strand of DNA.

### BACKGROUND OF THE INVENTION

In biotechnology, delivery vehicles for the transmission of nucleic acids (primarily DNA) are called vectors. Several DNA constructs or vectors, which are suitable for the expression of proteins, which are encoded by sequences within such a DNA construct, are known in the state of the art. Depending on the organism or cell type into which an expression construct should be introduced they are designated eukaryotic or prokaryotic expression constructs. The term "plasmid" is commonly related to circular expression constructs comprising a covalently closed circular DNA double-strand. Plasmids usually contain further sequences in addition to the ones, which should be expressed, like marker genes for their specific selection and in some cases sequences for their episomal replication in a target cell. Thus, alongside the genetic information required for the action, the vectors exhibit a large number of other structures, which are necessary for such processes as the production or augmentation of the vectors, e.g. resistance genes against antibiotics, but can engender significant drawbacks or adverse effects during treatment.

WO 2006081831 describes a method for producing expressionally and translationally active linear DNA molecules that can be used as protein expression cassettes and are useful for high throughput protein expression and analysis in living cells. The method comprises a PCR amplification using two primers complementary to the sequences flanking the DNA sequence of interest, such as a cDNA, an open reading frame or a gene, that exists in expression vectors.

The resultant PCR product contains a promoter, the DNA sequence of interest, and a termination sequence. The invention further provides different uses of these translationally active DNA cassettes, such as in proteome arrays.

Schakowski F et al (in: A Novel Minimal-Size Vector (MIDGE) Improves Transgene Expression in Colon Carcinoma Cells and Avoids Transfection of Undesired DNA Molecular Therapy (2001) 3, 793-800) describe that viral and plasmid vectors may cause unwanted immunological side effects resulting from the expression of nontherapeutic genes contained in their sequence. Furthermore, replication-defective viral vectors carry the potential risk of recombination with wild-type viruses or activation of oncogenes. They propose a new vector type for minimalistic, immunologically defined gene expression (MIDGE) that may overcome these problems. MIDGE is described as a minimal-size gene transfer unit containing the expression cassette, including promoter, gene, and RNA-stabilizing sequence, flanked by two short hairpin oligonucleotide sequences. The resulting vector is a small, linear, covalently closed, dumbbell-shaped molecule. DNA not encoding the desired gene is reduced to a minimum. They transfected colon carcinoma cell lines using cationic lipid, cationic polymer, and electroporation with several MIDGE vectors and corresponding plasmids containing transgenes encoding enhanced green fluorescent protein (eGFP) and human interleukin-2 (hIL-2). Transfection efficiency as measured qualitatively and quantitatively with eGFP was found to be comparable for both vector types. However, hIL-2 secretion and eGFP expression were approximately two- to fourfold higher in most cells transfected with these transgenes using MIDGE vectors compared to the plasmid control. This report demonstrates the advantages of this new vector type and its prospects for *ex vivo* gene therapy studies.

The MIDGE vector expression construct is also described in the EP 0 941 318. This document discloses a dumbbell-shaped linear, covalently closed DNA construct with partially single stranded loops at both ends. Such a construct causes after transfection significantly increased levels of protein expression, depending on the method for introducing the DNA construct into the cell. EP 0 941 318 discloses further that it is advantageously with regard to the protein expression to apply such a dumbbell-shaped DNA constructs via ballistic transfer. Although the expression level increases, it is laborious to link the DNA by means of absorption, covalently or ionic binding to the micro projectiles which are used for the ballistic transfer.

Endman et al (in Endmann A, Baden M, Weisermann E, Kapp K, Schroff M, Kleuss C, Wittig B, Juhls C. Immune response induced by a linear DNA vector: influence of dose, formulation and route of injection Vaccine. 2010 May 7;28(21):3642-9. Epub 2010 Mar 31) describe an evaluation of the influence of dose, formulation and delivery route on the immune response after vaccination with MIDGE-Th1 vectors encoding hepatitis B virus surface antigen (HBsAg). An HBsAg-specific IgG1 and IgG2a antibody response was induced in a dose-dependent manner, whereas the IgG2a/IgG1 ratio was independent of the injected DNA dose. Formulation of MIDGE-HBsAg-Th1 with the cationic pyridinium amphiphile SAINT-18 significantly increased antibody levels of IgG1 and IgG2a compared to the unformulated vector. The strongest immune response was generated after intradermal injection, followed by intramuscular and subcutaneous (s.c.) injection. The results show that the formulation of MIDGE-Th1 with SAINT-18 increased the efficacy of the MIDGE-Th1 DNA vaccine and is therefore a suitable approach to improve the efficacy of DNA vaccines also in large animals and humans.

Physical transfection methods, which are appropriate for nucleic acid, e.g. DNA, transfer into a cell or tissues, are known in the state of the art, e.g. for the application of vaccines. Such transfection methods comprise the acceleration of particles coated with a DNA expression construct, or microinjection techniques, such as a jet injector, which is usually a medical device with an injecting syringe, which uses a high-pressure narrow jet for the injection of liquids. The difference to usually used hypodermic needles to penetrate the epidermis is that a jet injector reduces the pain associated with the corresponding injection. Commonly, a jet injector is powered by compressed air or gas, either by a pressure hose from a large cylinder or from a built in gas cartridge or small cylinder. Basically, jet injectors are used as alternatives to needle syringes.

### BRIEF SUMMARY OF THE INVENTION

In view of the above, it is an object of the present invention to provide an improved use of linear DNA expression constructs having covalently closed ends, such as, for example, dumbbell-shaped DNA expression constructs, in the gene therapy-treatment of cancer.

In a first aspect of the present invention, said object is solved by a linear DNA expression construct having covalently closed ends, comprising an expression cassette comprising a coding sequence encoding for a gene therapy anti-cancer agent for use in the treatment of cancer, wherein said DNA expression construct is suitable for administration into cells or tissues by a physical transfection method.

According to the present invention, the physical transfection method preferably comprises the acceleration of particles coated with said DNA expression construct, or microinjection techniques. Other physical transfection methods comprise the use of pressure, electroporation, silica/carbon fibers, laser mediated transfection, and sonication (SAT).

It is intended that the linear DNA expression construct as used according to the present invention comprises an expression cassette which comprises a minimum of foreign DNA sequences in addition to the coding sequence. In the context of the present invention, the term "foreign DNA sequences" shall mean all DNA sequences that are part of the expression cassette and/or the expression construct and are not derived from the organism to be transformed, such as, for example, the patient to be treated. Particular examples for foreign DNA sequences are sequences of viral or bacterial origin.

According to the present invention, a preferred expression cassette comprises a promoter sequence, a coding sequence and a termination signal. The promoter sequence is preferably operable in vitro and/or in vivo in eukaryotic cells, such as, for example, in mammalian cells, such as, for example, human cells and tissues. It might be advantageous to adapt the used promoter to the tissue or the origin of the respective tissue, in order to optimize the expression rate after transfer into the tissue, cell or nucleus, respectively.

In order to protect the linear DNA expression construct from degradation by intracellular nucleases and, therefore, enhance expression and translation of the cassettes, one or both of the 5' ends may be modified by any method that protects these ends. There is a variety of methods available, for example, as part of the primer synthesis services by a number of companies, such as Sigma-GenoSys, prooligo, Qiagen, MWG, and many others. Such modifications include but are not limited to phosphorylation, amino group, cholesterol moieties, peptide nucleic acids (PNA), and hairpin structures. Preferred is the linear DNA expression construct according to the present invention, wherein the covalently closed ends are single-stranded loops located at both ends of a double-stranded stem. To generate the DNA with a minimum of foreign DNA sequences, one strategy is to use the prokaryotic telomerase, protelomerase TelN, of bacteriophage N15. This is an enzyme with cleaving-joining activity, which is required for the formation of linear prophage DNA with closed ends in lysogenic bacteria. Acting on a telomere resolution site telRL, the protelomerase converts circular plasmid DNA into linear covalently closed dumbbell-shaped molecules ("doggybones") in a single-step enzyme reaction.

In another preferred linear DNA expression construct according to the present invention, the hairpin structures can be chemically altered. Because of this, modifications with peptides, carbohydrates and many other elements can be implemented. The DNA expression constructs generated in this way can be used in a variety of medical applications as well as customized for research, and are especially well suited for the development of vaccines.

The linear DNA constructs can be used to encode immunomodulating agents and/or cell surface marker. The term "immunomodulator" comprises stimulators and suppressors of the immune system. The term "modulation of the immune system" is used synonymously with "activation of the immune system" either in the meaning of intensifying or suppressing an immune response.

According to the invention, the immunomodulating agents shall be selected from the group consisting of antibodies, hormones, cytokines or other biologically active substances, such as, for example, siRNA. The term "biologically active" comprises all substances, which cause an effect after their application or transfer into cells or tissues, wherein it does not matter whether the respective substance is synthetically produced or of biological origin, which includes genetically based methods of manufacture. Preferred is a DNA construct encoding transmitters or messengers of the immune system, such as, for example, cytokines, which are selected from the group comprising TNF-α, Interleukin-7, granulocyte-macrophage colony stimulating factor, CD40L/CD154 and B7.1/CD80. Besides the mentioned transmitters, any other molecule mediating an immunological signal is within the scope of the present invention.

In another aspect of the present invention, the linear DNA expression construct is administered simultaneously or subsequently with a chemotherapeutic agent, wherein a simultaneous administration includes the option that one compound is released with a time-delay due to a corresponding formulation or coupling to adequate compounds. The chemotherapeutic agent can be selected from the group consisting of antibodies, alkylating agents, platinum analoga, intercalating agents, antibiotics, mitosis suppressors, taxanes, topoisomerase suppressors, anti-metabolites and/or L-asparaginase, hydroxycarbamide, mitotanes, and/or amanitines.

Preferred is a mitosis suppressor mitosis selected from a vinca alkaloid, such as, for example, vindesine vinblastine, vincristine, and vinorelbine or a third-generation bifluorinated semisynthetic vinca alkaloid, such as vinflunine. Particulalry preferred is vindesine, which showed very good results in combination with a DNA expression construct according to the present invention encoding for TNF-α.

It is further intended that the linear DNA expression construct is provided/administered as pharmaceutically applicable composition, especially a vaccine.

In another aspect of the present invention, the linear DNA expression construct is further coadministered with a non-coding, immunomodulatory DNA sequence, preferably by jet injection. Again, this additional compound can be administered simultaneously or subsequently and even in the case of simultaneous administration a time-delayed release can be intended. The non-coding DNA sequence can also be included in the same linear DNA expression construct

It is intended that the DNA sequence of said non-coding DNA sequence comprises at least one sequence motif N¹N²CGN³N⁴ wherein N¹N² is a sequence from the group of GT, GG, GA, AT, and AA, and N³N⁴ is a sequence from the group of CT and TT, and C is deoxycytosine, G is deoxyguanosine, A is deoxyadenosine, and T is deoxythymidine.

In case that the non-coding is an open-chained oligodeoxynucleotide, suitable means for protection of the ends against nucleases (e.g. modified nucleotides) are within the scope of the invention.

The non-coding immunomodulatory DNA can be administered in the form of a linear DNA construct with covalently closed ends, preferably as a dumbbell-shaped construct as discussed above for the coding sequence, and most preferably comprise a covalently closed circular DNA single strand, which is partly double-stranded forming a double-stranded stem and single stranded loops at both ends of the stem.

Another aspect of the present invention the relates to a method of treating cancer, comprising administering to a patient in need of said treatment a linear DNA expression construct according to the present invention as described herein, optionally together with a non-coding, immunomodulatory DNA sequence according to the present invention as described herein.

The present invention provides a novel and improved use of a DNA construct, which shall be administered by a physical transfection method, e.g. jet injection, into cells or tissues. On one hand, the DNA may be transferred or injected directly into tissues like tumorous tissues, or muscle or connective tissues. On the other hand, the use according to the invention may be performed with cell cultures or tissues cultures. Thus, the present invention provides both in vivo or in vitro uses.

The scope of the present invention covers not only dumbbell-shaped expression constructs, but also circular double-stranded DNA expression constructs. Such an expression construct might encode TNF-α and should be applied together with a chemotherapeutic, like vindesine.

According to the present invention, the term "cancer" shall include malign proliferative diseases, such as cancerous diseases, or tumors that are treated (i.e. reversed progression, or slowed down progression) or prevented, wherein said cancer is selected from the group consisting of mammary carcinomas, melanoma, skin neoplasms, gastrointestinal tumors, including colon carcinomas, stomach carcinomas, pancreas carcinomas, colon cancer, small intestine cancer, ovarian carcinomas, cervical carcinomas, lung cancer, prostate cancer, kidney cell carcinomas, head/neck cancer, esophageal cancer, and/or liver cancer; and respective metastases. Most preferred is skin cancer, such as, for example, melanoma.

The pharmaceutical composition as used according to the present invention shall be preferably suitable to be used in physical transfection methods, such as, for example, injection, especially jet injection, including sterile aqueous solutions or dispersions and sterile powders for the extemporaneous preparation of sterile injectable solutions or dispersions. In all cases the pharmaceutical composition shall be provided as sterile fluid in an extent that easy syringability or jet injection is possible. It has to be stable under the conditions of manufacture and storage and must be preserved against the contaminating action of microorganisms, such as bacteria and fungi. The carrier can be a solvent or dispersion medium containing, for example, water, ethanol, polyol (e.g., glycerol, propylene glycol, and liquid polyethylene glycol, and the like), suitable mixtures thereof, and/or vegetable oils. Proper fluidity may be maintained, for example, by the use of a coating, such as lecithin, by the maintenance of the required particle size in the case of dispersion and by the use of surfactants. The prevention of the action of microorganisms can be facilitated by various antibacterial and antifungal agents, for example, parabens, chlorobutanol, phenol, sorbic acid, thimerosal, and the like. In many cases, it will be preferred to include isotonic agents, for example, sugars or sodium chloride. Prolonged absorption of the injectable compositions can be brought about by the use in the compositions of agents delaying absorption, for example, aluminum monostearate and gelatin.

Sterile injectable solutions are prepared by incorporating the active compound(s) in the required amount in the appropriate solvent with several other ingredients as described above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques, which yield a powder of the active ingredient plus any additional desired ingredient from a previously sterile-filtered solution thereof.

The pharmaceutical compositions as used herein may be formulated in a neutral or salt form. Pharmaceutically-acceptable salts include the acid addition salts (formed with the free amino groups of the protein) and are formed with inorganic acids such as, for example, hydrochloric or phosphoric acids, or such organic acids as acetic, oxalic, tartaric, mandelic, and the like. Salts formed with the free carboxyl groups can also be derived from inorganic bases such as, for example, sodium, potassium, ammonium, calcium, or ferric hydroxides, and such organic bases as isopropylamine, trimethylamine, histidine, procaine, and the like. Upon formulation, Solutions will be administered in a manner compatible with the dosage formulation and in such amount as is therapeutically effective. The formulations are easily administered in a variety of dosage forms, such as injectable solutions, drug-release capsules, and the like.

As used herein, "carrier" includes any and all solvents, dispersion media, vehicles, coatings, diluents, antibacterial and antifungal agents, isotonic and absorption delaying agents, buffers, carrier Solutions, suspensions, colloids, and the like. The use of such media and agents for pharmaceutical active substances is well known in the art. Except insofar as any conventional media or agent is incompatible with the active ingredient, its use in the therapeutic compositions is contemplated. Supplementary active ingredients can also be incorporated into the compositions.

The term "pharmaceutically acceptable" refers to molecular entities and compositions that do not produce an allergic or similar untoward reaction when administered to a human. The preparation of an aqueous composition that contains a protein as an active ingredient is well understood in the art. Typically, such compositions are prepared as injectables, either as liquid solutions or suspensions; solid forms suitable for solution in, or suspension in, liquid prior to injection can also be prepared. The preparation can also be emulsified.

The invention will be further illustrated by figures and examples without being limited to these. For the purposes of the present invention, all references as cited herein are incorporated by reference in their entireties. The Figures show:
Figure 1: Dose dependency of dumbbell-shaped DNA jet injected into melanomas.
Figure 2: *In vivo* expression efficiency in tumors comparing dumbbell-shaped DNA with circular double-stranded plasmids.
Figure 3: Gene transfer using dumbbell-shaped DNA alone or in combination with vindesine.

Although the following results have been obtained using a dumbbell shaped DNA construct encoding TNF-α, the person of skill, upon reading the following disclosure, will be readily able to adapt the disclosure to other expression constructs according to the present invention encoding other gene therapy-agents, such as, for example, siRNA(s).

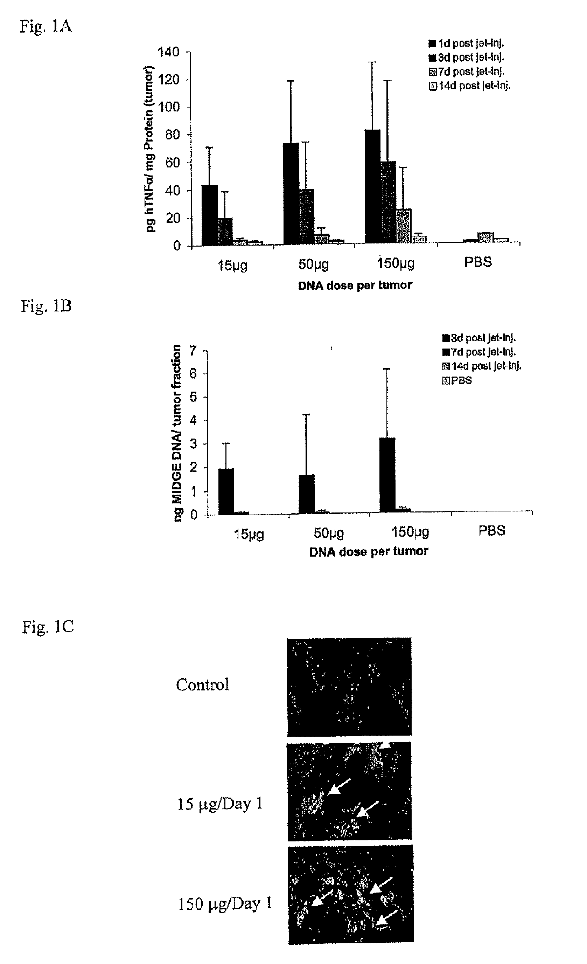

Figure 1 shows the dose dependency of the protein expression and the presence of transferred DNA from the respective dose applied to the tumor. The transferred dumbbell shaped DNA construct encoded TNF-α. Three different methods were applied to demonstrate the expression of TNF-α or the presence of the transferred DNA in melanomas, which are malignant tumors of melanocytes.

Figure 1A shows the results of a TNF-ELISA with the applied DNA dose depicted on the x-axis (pg) in relation to the amount of TNF-α (pg) per amount of total protein (mg) on the y-axis for days one, three, seven and 14 after jet injection of the DNA.

It is obvious that the level of protein expression after jet injection depends on the DNA dose per tumor. For each time point after jet injection the protein expression has the highest level for 150 pg DNA. The strongest DNA expression can be observed one day after jet injection of 150 pg DNA and decreases for each further time point measured. On the right side of Figure 1A PBS without any DNA is shown as negative control.

Figure 1B shows the amount of transfected DNA construct (ng) per tumor fraction on the y-axis for different DNA doses for days three, seven and 14 after jet injection of the DNA. The DNA of the transfected dumbbell-shaped DNA construct was amplified by PCR and PBS was used as negative control, which is depicted on the right side of the x-axis.

The results of the DNA amplification coincide with the results of the protein determination in Figure 1A. The highest level of DNA could be amplified three days after jet injection and a minimal amount of DNA could be determined seven days after jet injection.

Although the DNA dose of 50 pg per tumor results in less DNA expression than with 15 pg DNA, the error bar shows that the average DNA expression with 50 pg DNA was higher than with 15 pg. In total, the highest DNA dose of 150 pg per tumor resulted in the highest amount of transferred DNA, which was amplifiable by PCR.

Figure 1C shows an immunofluorescence for the determination of TNF-α expression in tumors. The arrows in the figures are depicting cells expressing TNF-α. It is obvious that the transfer of 15 pg and 150 pg results one day after jet injection in a clearly detectable expression of TNF-α. The picture on top shows the negative control.

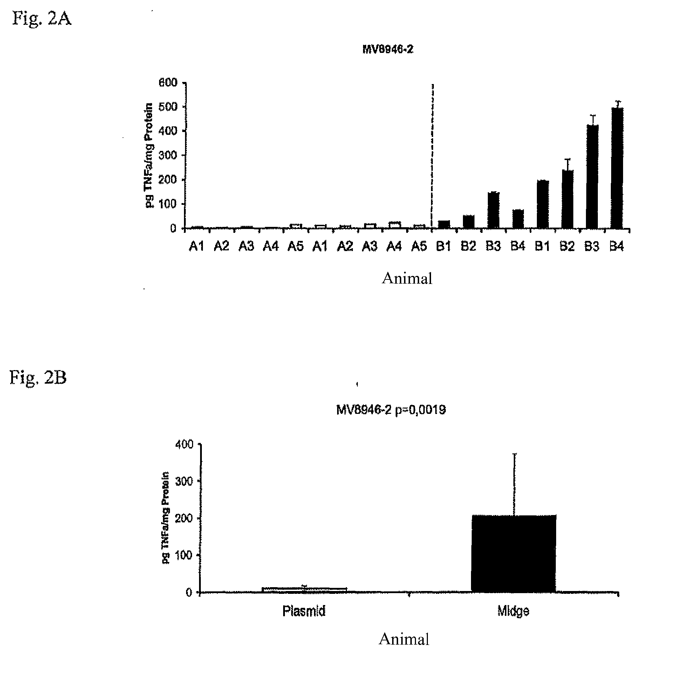

Figure 2 shows the in vivo expression efficiency in tumors comparing dumbbell-shaped DNA with circular closed double-stranded DNA. Both DNA expression constructs encode TNF-α. In Figure 2A and B the results for the plasmid DNA - double-stranded circular DNA - are depicted on the left side (open bars) in comparison to the results of the dumbbell-shaped DNA construct on each right side (black bars). Equimolar amounts of the respective DNA construct were applied.

Five different animals for the plasmid transfer and four different animals for the dumbbell-shaped DNA were used for the experiments and the weight of the tumors together with the mass of TNF (pg) per mass tumor (g) is indicated in the following table 1.

**Table 1:**

| Animal # | Tumor weight | pgTNF/g tumor |
|---|---|---|
| 1 | 0.6 g | 7.005 |
| 2 | 1.25 g | 12.189 |
| 3 | 1.27 g | 6.567 |
| 4 | 0.62 g | 5.656 |
| 5 | 0.5 g | 6.898 |

Figure 2A shows the amount of TNF (pg) per mass of total protein (mg). It is obvious that the use of plasmid DNA did not result in a measurable amount of expressed TNF-α (left side), wherein the use of the dumbbell-shaped DNA construct resulted in a clearly detectable amount of expressed TNF-α (right side).

As it can be taken from Figure 2B the average amount of TNF-α per total protein mass is about 200 fold more using the dumbbell-shaped DNA construct instead of plasmid DNA. The differences between the expression efficiencies for plasmid DNA versus dumbbell-shaped DNA are absolutely surprising and could not be expected, even when taking into account that jet injection might result in a better transfer of DNA constructs. Although the plasmid DNA was transferred by jet injection, it is nearly no TNF-α expression detectable, whereas the dumbbell-shaped DNA caused a considerable amount of expressed TNF-α.

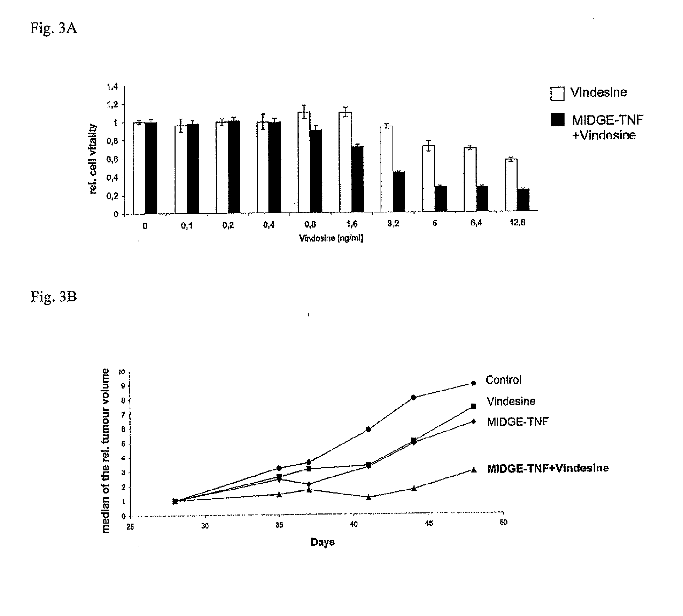

In further experiments the effect of TNF-α expression on cell vitality and tumor volume was investigated. Figure 3 shows the results of a gene transfer using a dumbbell-shaped DNA construct encoding TNF-cc in combination with the chemotherapeutic vindesine (black bars) in comparison to the use of vindesine alone (open bars).

Figure 3A shows the results of an in vitro chemo-sensitization assay of A375 melanoma cells after the transfer of vindesine alone and in combination with a dumbbell-shaped DNA construct encoding TNF-α. It is clearly visible that the use of vindesine alone decreases cell vitality. This effect can be amplified by using a combination of vindesine with the dumbbell-shaped DNA construct encoding TNF-α. Using vindesine alone in a concentration of 12.8 pg per ml results in a relative cell vitality of 0.6 in comparison to 0.2 when using in parallel the dumbbell-shaped construct encoding TNF-α. The degree of amplification of the effect is surprising and could not be traced back to the addition of the effect of the TNF-α encoding DNA construct and vindesine, respectively, as can be taken from Figure 3B.

Figure 3B shows the chemo-sensitization of A375 melanoma xenotransplanted tumors by the gene transfer of the dumbbell-shaped DNA construct encoding TNF-α (MIDGE-TNF), vindesine and the combination of MIDGE-TNF with vindesine. The intra-tumoral j et injection was performed using twice 150 pg of the dumbbell-shaped DNA construct and where indicated additionally twice 0.5 mg/kg tumor vindesine.

On the y-axis is the median of the relative tumor volume shown for the indicated days after transfer (x-axis). The transfer of vindesine and linear DNA expression construct (MIDGE-TNF), respectively, reduces the average tumor volume, but the combination of vindesine and MIDGE-TNF has a clear synergistic effect, which is far beyond the simple addition of the effects when using the compounds alone. This result could not be expected and strengthens the hypothesis that the cell vitality (Fig. 3A) is also decreased by a synergistic effect when vindesine is applied by jet injection in combination with the TNF-α encoding dumbbell-shaped construct.

The figures show that a dumbbell-shaped DNA construct, which is applied by jet injection, leads to a multiple expression of encoded protein in comparison to jet injected plasmid DNA. The use of the dumbbell-shaped DNA construct in combination with a chemotherapeutic - like vindesine - has a synergistic effect on the tumor cell vitality and the tumor volume. Both effects could not be expected because they are much stronger than simply adding the effects of the compounds alone.

In general, the above results show that the use of a linear DNA expression construct according to the present invention, e.g. the dumbbell-shaped DNA construct encoding for immunomodulators, namely immunostimulators or immunosuppressors, in combination with a chemotherapeutic and applying the DNA by jet injection is advantageously for the treatment of cancer or any proliferative disease, which is related to uncontrolled or malign cell growth.

## Claims

1. A linear DNA expression construct having covalently closed ends, comprising an expression cassette comprising a coding sequence encoding for a gene therapy anti-cancer agent for use in the treatment of cancer, wherein said DNA expression construct is suitable for administration into cells or tissues by a physical transfection method.

2. The linear DNA expression construct according to claim 1, wherein said physical transfection method comprises the acceleration of particles coated with said DNA expression construct, or microinjection techniques.

3. The linear DNA expression construct according to claim 1 or 2, wherein the expression cassette comprises a minimum of foreign DNA sequences in addition to the coding sequence.

4. The linear DNA expression construct according to any of claims 1 to 3, wherein the expression cassette comprises a promoter sequence, a coding sequence and a termination signal.

5. The linear DNA expression construct according to any of claims 1 to 4, wherein the covalently closed ends are single-stranded loops located at both ends of a double-stranded stem.

6. The linear DNA expression construct according to any of claims 1 to 5, wherein the promoter sequence is operable in vitro and/or in vivo in eukaryotic cells, such as, for example, in mammalian cells.

7. The linear DNA expression construct according to any of claims 1 to 6, wherein the coding sequence encodes for immunomodulating agents, such as, for example, selected from the group comprising antibodies, hormones, cytokines or other biologically active substances; siRNA; and/or a cell surface marker.

8. The linear DNA expression construct according to claim 7, wherein the cytokines are selected from the group comprising TNF-α, Interleukin-7, granulocyte-macrophage colony stimulating factor, CD40L/CD154, and B7.1/CD80.

9. The linear DNA expression construct according to any of claims 1 to 8, wherein said DNA construct is administered simultaneously or subsequently with a chemotherapeutic agent, such as, for example, selected from the group comprising antibodies, alkylating agents, platinum analoga, intercalating agents, antibiotics, mitosis suppressors, taxanes, topoisomerase suppressors, anti-metabolites and/or L-asparaginase, hydroxycarbamide, mitotanes, and/or amanitines.

10. The linear DNA expression construct according to claim 9, wherein the mitosis suppressor is selected from a vinca alkaloid, such as, for example, vindesine vinblastine, vincristine, and vinorelbine or a third-generation bifluorinated semisynthetic vinca alkaloid, such as vinflunine.

11. The linear DNA expression construct according to any of claims 1 to 10, wherein the DNA construct is provided as pharmaceutically applicable composition, such as, for example, a vaccine.

12. The linear DNA expression construct according to claim 11, wherein additionally a non-coding, immunomodulatory DNA sequence is administered.

13. The linear DNA expression construct according to claim 12, wherein the DNA sequence comprises at least one sequence motif N¹N²CGN³N⁴ wherein N¹N² is a sequence from the group of GT, GG, GA, AT, and AA, and N³N⁴ is a sequence from the group of CT and TT, and C is deoxycytosine, G is deoxyguanosine, A is deoxyadenosine, and T is deoxythymidine.

14. The linear DNA expression construct according to claim 12 or 13, wherein said non-coding immunomodulatory DNA sequence is administered in the form of a linear DNA construct with covalently closed ends.

15. A method of treating cancer, comprising administering to a patient in need of said treatment a linear DNA expression construct according to any of claims 1 to 11, optionally together with a non-coding, immunomodulatory DNA sequence according to any of claims 12 to 14.
